Europäisches Patentamt

European Patent Office

Office européen des brevets

(19)

(11) Veröffentlichungsnummer: **0 046 970**
**B2**

(12) **NEUE EUROPÄISCHE PATENTSCHRIFT**

(45) Veröffentlichungstag der neuen Patentschrift:
06.04.88

(21) Anmeldenummer: **81106582.0**

(22) Anmeldetag: **25.08.81**

(51) Int. Cl.⁴: **C 07 H 13/08,** C 07 C 69/753, A 61 K 7/00, C 07 C 137/00, C 07 H 13/06

(54) Gewünschtenfalls mit Natriumsulfit umgesetzte Partialester von mehrwertigen Alkoholen, Verfahren zu ihrer Herstellung und ihre Verwendung als hautfreundliche, nicht ionogene und/oder anionenaktive oberflächenaktive Substanzen.

(30) Priorität: 29.08.80 DE 3032612

(43) Veröffentlichungstag der Anmeldung:
10.03.82 Patentblatt 82/10

(45) Bekanntmachung des Hinweises auf die Patenterteilung:
28.03.84 Patentblatt 84/13

(45) Bekanntmachung des Hinweises auf die Entscheidung über den Einspruch:
06.04.88 Patentblatt 88/14

(84) Benannte Vertragsstaaten:
AT BE CH DE FR GB IT LI LU NL SE

(56) Entgegenhaltungen:
DE-B-1 178 585
DE-B-1 202 002
GB-A-1 551 894
US-A-2 721 505
US-A-3 016 393
US-A-3 098 065
US-A-3 985 504

Chem. Abstr. 42 (1948) 3803h

Die Akte enthält technische Angaben, die nach dem Eingang der Anmeldung eingereicht wurden und die nicht in dieser Patentschrift enthalten sind.

(73) Patentinhaber: Grillo- Werke AG, Weseler Strasse 1, D-4100 Duisburg- Hamborn (DE)

(72) Erfinder: Lowicki, Norbert, Dr. Ing. Dipl.- Chem., Walramsweg 26, D-4100 Duisburg 1 (DE)
Erfinder: Desai, Natvarial B, Dr. rer. nat. Dipl.- Chem., Margaretenweg 35, D-4220 Dinslaken (DE)

(74) Vertreter: Werner, Hans- Karsten, Dr., Deichmannhaus am Hauptbahnhof, D-5000 Köln 1 (DE)

EP 0 046 970 B2

## Beschreibung

Gewünschtenfalls mil Natriumsulfit umgesetzte Partialester von mehrwertigen Alkoholen, Verfahren zu ihrer Herstellung und ihre Verwendung als hautfreundliche, nichtionogene und/oder anionenaktive oberflächenaktive Substanzen.

Die Erfindung betrifft Partialester von Di- oder Polyhydroxyalkalen mit 2 oder 3 Kohlenstoffatomen oder Partialester von Mono- und Disacchariden mit den mehrwertigen Carbonsäuren, welche aus den En-Addukten von Maleinsäureanhydrid mit mindestens einfach ungesättigten Carbonsäuren mit einer Kettenlänge von $C_{10}$ bis $C_{25}$ entstehen und welche gewünschtenfalls mit Natriumsulfit umgesetzt sein können. Weiterhin betrifft die Erfindung Verfahren zur Herstellung dieser Produkte sowie ihre Verwendung als hautfreundliche, nichtionogene und/oder anionenaktive oberflächenaktive Substanzen in kosmetischen Präparaten.

Die En-Addukte von Maleinsäureanhydrid mit mindestens einfach ungesättigten Carbonsäuren sind bekannt. Sie können mit Alkali verseift werden und ihre Ester und Amide, insbesondere ihre Umsetzungsprodukte mit Triäthanolamin sind als Korrosionsschutzmittel bekannt geworden; vgl. US-PS Nr. 3 985 504.

Die Alkalisalze sind auch schon zur Verhinderung von Ablagerungen von heissem Meereswasser verwendet worden; vgl. GB-PS Nr. 1 551 894. Die chemische Struktur der Addukte scheint noch nicht völlig geklärt zu sein, da sich die Angaben hierüber in den beiden obengenannten Patentschriften widersprechen. Gemäss US-PS Nr. 3 985 504 findet eine Anlagerung von Kohlenstoffatomen neben der Doppelbindung statt, wobei die Doppelbindung erhalten bleibt. Gemäss GB-PS Nr. 1 551 894 entstehen Cyclobutandicarbonsäuren. Auf alle Fälle entstehen durch Hydrolyse der Addukte drei- und mehrwertige Carbonsäuren.

Die Umsetzung von einfach ungesättigten Carbonsäuren mit Maleinsäureanhydrid wurde zunächst auch als Diels-Alder-Reaktion bezeichnet. Nach neuerer Nomenklatur ist die Bezeichnung Diels-Alder-Reaktion beschränkt auf die Umsetzung eines Diens mit einem Dienophil. Die Umsetzung einfach ungesättigter Verbindungen mit einem Dienophil wird nach neuerer Nomenklatur En-Reaktion genannt; vgl. "Liebig's Annalen" 651, S. 141 (1962). Das En-Addukt aus Maleinsäureanhydrid und einer einfach ungesättigten Fettsäure enthält immer noch eine Doppelbindung. Diese Doppelbindung bleibt auch erhalten bei einer anschliessenden Umsetzung mit Natriumsulfit.

Die DE-AS-11 78 585 betrifft ein Verfahren zur Herstellung von Urethangruppen aufweisenden Schaumstoffen auf Grundlage von mit Hilfe der Diels-Alder-Reaktion aus ein- und mehrwertigen Carbonsäuren sowie mehrwertigen Alkoholen hergestellten, gegebenenfalls halogenierten Polyhydroxylverbindungen mit mehr als zwei Hydroxylgruppen, Polyisocyanaten sowie Wasser und/oder anderen Treibmitteln, bei dem man gegebenenfalls halogenierte Polyhydroxylverbindungen mit mehr als zwei Hydroxylgruppen verwendet, die durch Diels-Alder-Rekation einer ungesättigten acyclischen einwertigen, natürlich vorkommenden Fettsäure mit mindestens 0,2 Mol einer $\alpha,\beta$-ungesättigten Polycarbonsäure und Veresterung des Adduktes mit einem mehrwertigen Alkohol, gegebenenfalls unter Mitverwendung einer gesättigten Polycarbonsäure, erhalten worden sind, wobei die so erhaltenen Polyhydroxylverbindungen auch mit anderen bekannten Polyhydroxylverbindungen gemischt sein können. Bei diesem Verfahren sind die erfindungsgemäßen Produkte nicht einmal als Zwischenprodukte entstanden, da hier nicht zuvor mit Wasser zu den Carbonsäuren hydrolysiert und erst danach bei 130 bis 140°C mit den Di- oder Polyhydroxyalkanen verestert worden ist. Weiterhin wurde bereits die Kondensation in Gegenwart eines Titankatalysators durchgeführt.

Die US-A-2 721 505 beschreibt ein Verfahren zum Sprühbeschichten von fasrigem Material, bei welchem als Bindemittel bzw. Imprägniermittel ein Produkt eingesetzt werden soll, das durch Umsetzung von Tallöl mit Maleinsäureanhydrid und anschließende vollständige Veresterung mit Glycerin oder Pentaerythrit entstanden ist. Auch dieses Zwischenprodukt ist mit Sicherheit kein Partialester, sondern ein vollständiger Ester und ist somit mit den erfindungsgemäßen Produkten nicht vergleichbar.

Die DE-AS-12 02 002 beschreibt ein Verfahren zur Herstellung von Polyethylenglykolestern mit hohem Molekulargewicht, welche als Reinigungsmittel in hartem Wasser geeignet sind. Es handelt sich um relativ langkettige Ätherester, bei denen nur die Anhydridgruppe unter Bildung eines Monoesters mit zwei freien Carboxylgruppen reagiert hat. Die Produkte weisen so viele freie Carboxylgruppen auf, daß sie deutlich sauer reagieren. Sie sind deshalb auch nur für saure Reinigungen geeignet, wie die Reinigung von Filzen und Vliesen im Rahmen der Papierfabrikation.

Die US-A-3 016 393 beschreibt oberflächenaktive sulfonierte Polyester, welche durch Umsetzung von 2 Mol Ethylenglykol und 1 Mol Maleinsäureanhydrid entstehen und welche anschließend mit 2 Mol Fettsäuren zu gemischten Estern umgesetzt werden. Diese ungesättigten gemischten Polyester werden dann anschließend mit wässrigem Bisulfit sulfoniert. Im Beispiel IV werden Maleinsäureanhydrid, Propylenglykol und ungesättigte Fettsäuren gleichzeitig miteinander erhitzt. Sofern sich hierbei in dem Gemisch auch Komponenten gebildet haben sollten, die in dem erfindungsgemäßen Reaktionsgemisch als Zwischenprodukte entstehen, so unterscheiden sich diese unter anderem dadurch, daß die Kondensation in Gegenwart eines Lösungsmittels stattgefunden hat, welches auch mit dem Kondensationsprodukt reagieren kann. Keinesfalls erfolgt vor der Veresterung eine Hydrolyse zu den freien Carbonsäuren. Die Endprodukte der dort beschriebenen Gemische sind weiterhin keine Partialester, sondern vollständig veresterte Gemische. Nach der Umsetzung mit Bisulfit sind deshalb sicherlich auch andere Produkte entstanden als beim erfindungsgemäßen Verfahren. Keine der in diesem Stand der Technik beschriebenen Produkte hat die überraschend guten Eigenschaften als Rohstoff für Körperpflegemittel aufgewiesen.

2

Es wurde jetzt gefunden, daß bestimmte Partialester von Di- oder Polyhydroxyalkanen mit 2 oder 3 Kohlenstoffatomen oder Partialester von Mono- und Disacchariden mit den En-Addukten von Maleinsäureanhydrid an mindestens einfach ungesättigte Carbonsäuren mit einer Kettenlänge von $C_{10}$ bis $C_{25}$ ungewöhnliche und außerordentlich wertvolle Eigenschaften aufweisen. Die Erfindungsgemäßen Partialester sind erhältlich durch Umsetzung der Carbonsäuren mit Maleinsäureanhydrid ohne Lösungsmittel und ohne Katalysator bei Temperaturen von 150 bis 200°C innerhalb von 0,5 bis 5h in Stickstoffatmosphäre, Hydrolyse der so erhaltenen En-Addukte und anschließende Veresterung mit den Di- oder Polyhydroxyalkanen bei 130 bis 140°C bis zu einer Säurezahl von höchstens 140, woraufhin gewünschtenfalls bei 70 bis 100°C mit den Mono- oder Disacchariden umgesetzt und gewünschtenfalls bei 70 bis 80°C nachträglich mit Natriumsulfit umgesetzt wird.

Es wurde gefunden, dass diese Partialester hervorragend hautfreundliche nichtionogene oberflächenaktive Substanzen darstellen, die insbesondere für kosmetische Präparate geeignet sind. Überraschenderweise lassen sich diese Partialester unter schonenden Bedingungen mit Natriumsulfit umsetzen, wobei die Doppelbindung der ungesättigten Carbonsäuren offensichtlich unverändert bleibt, was sich beispielsweise aus der Jodzahl ergibt. Die mit Natriumsulfit umgesetzten Partialester bilden ebenfalls ausserordentlich hautfreundliche anionenaktive oberflächenaktive Substanzen, die ebenfalls besonders für kosmetische Präparate geeignet sind.

Die mit Natriumsulfit umgesetzten Partialester mit Mono- und Disacchariden sind die ersten sulfierten Zuckerester und bieten sich daher besonders für milde Reinigungsmittel und hautfreundliche Waschmittel an.

Die Umsetzung mit Natriumsulfit kann bei so milden Bedingungen durchgeführt werden, nämlich bei Temperaturen von 70-80°C, dass es sich keinesfalls um die übliche Sulfierung von Olefinen handeln kann. Es wird daher angenommen, dass bei der Sulfierung der erfindungsgemässen Produkte eine Substitution an einem aktivierten C-Atom des Maleinsäuremoleküls stattfindet. Die erfindungsgemässen Partialester mit Mono- und Disacchariden stellen einen völlig neuen Typ von Zuckerestern und Zuckertensiden dar, die aufgrund ihrer günstigen toxikologischen und dermatologischen Eigenschaften allen bisher bekannten Produkten deutlich überlegen sind.

Ein besonderer Vorteil der erfindungsgemässen Ester ist die Verträglichkeit mit hartem Wasser, aus dem sie keine Ausfällungen erzeugen.

Weitere überraschende Vorteile ergeben sich bei der Herstellung der erfindungsgemässen Zuckerester, da sie sich besonders leicht und schonend im grosstechnischen Maßstab herstellen lassen. Gemäss "Industrial Engineering Chemistry", 48 (1956), S. 1459 bis 1464, werden Zuckerester beispielsweise durch Umesterung von Fettsäurealkylestern mit Zucker in Dimethylformamid unter Zusatz von alkalischen Katalysatoren bei ca. 90°C hergestellt. Die Reaktionszeit beträgt 12 h und die Reinigung des Reaktionsproduktes ist, besonders im Hinblick auf eine vollständige Entfernung des Dimethylformamids, schwierig. Die quantitative Rückgewinnung desselben ist schon aus wirtschaftlichen Gründen notwendig. Andererseits ist Dimethylformamid physiologisch nicht unbedenklich. Trotz sorgfältiger Reinigung des auf diese Weise gewonnenen Zuckeresters, beispielsweise Saccharoseesters, verbleiben geringe Mengen Dimethylformamid im Endprodukt, die physiologisch zu beanstanden sind und die ausserdem einen unangenehmen Geruch verursachen.

Gemäss DE-AS Nr. 2 022 880 kann die Umesterung von Fettsäureestern mit Saccharose durch Zusatz von 1 bis 40 Gew.-% alkalifreier Alkalisalze von gesättigten und ungesättigten Fettsäuren als Katalysator erfolgen. Die Umesterung wird bei 160 bis 190°C innerhalb von 20 min durchgefuhrt. Nach Beendigung der Reaktion wird das verbleibende Gemisch rasch abgekühlt und die nicht umgesetzte Saccharose sowie die als Katalysator verwendeten Seifen entfernt. Obwohl das Verfahren ohne Lösungsmittel arbeitet, ist der Aufwand, den man für die Herstellung der Alkaliseifen betreiben muss, sehr hoch. Um alkalifreie Seifen herzustellen, werden nämlich die Hydroxyde des Alkalimetalls in wasserfreiem Methanol unter Rückflüss durch Einleiten von Stickstoff in der Wärme gelöst, danach der Methylester der gewählten langkettigen Fettsäure zugegeben und das Gemisch 40 bis 60 min unter Rückflüss erhitzt. Das Methanol wird dann von der gebildeten Seife im Vakuum abdestilliert.

Ein weiterer Nachteil dieses Verfahrens ist darin zu sehen, dass Ausbeuten an Saccharoseester nur etwa 43 bis 48 % betragen und es schwierig ist, die hohen Reaktionstemperaturen so zu beherrschen, dass es zu keinen Zersetzungen kommt.

Gemäss DE-AS Nr. 2 412 374 kann man Saccharose mit einem Triglycerid bei 100 bis 170°C unter Verwendung alkalischer Katalysatoren umestern. Die Ausbeute an Saccharoseestern nach 10 bis 22 h Reaktionszeit liegt zwischen 11 und 40 %. Es entstehen hierbei feste Produkte, die in Wasser nicht oder nur sehr schwer löslich sind und die mit den Härtebildnern des Wassers reagieren.

Das Verfahren gemäss DE-AS Nr. 2 412 374 lässt sich nicht auf ungesättigte Fettsäuren übertragen, da man bei den langen Reaktionszeiten dunkle harzartige Produkte mit mangelhafter Dispergiereigenschaft in Öl erhält. Schliesslich ist die Aufarbeitung der in geringer Ausbeute anfallenden Saccharoseester sehr aufwendig.

Erfindungsgemäss erhält man dagegen Partialester von Mono- und Disacchariden, insbesondere auch der sehr empfindlichen Saccharose bei 85 bis 90°C innerhalb von 1 bis 5 h. Die so erhaltenen Produkte sind vollständig wasserlöslich und weisen von vornherein die hervorragenden Haut- und Schleimhautverträglichkeiten auf. Bei dem erfindungsgemässen Verfahren wird ohne Lösungsmittel gearbeitet und es genügen 5 bis 10 % Alkali, beispielsweise Natriumhydroxyd, Kaliumcarbonat oder Trinatriumphosphat als Katalysator. Überraschenderweise sinkt mit fortschreitender Reaktion die Viskosität des Reaktionsgemisches, während nach dem Stand der Technik die Reaktionsprodukte zunehmend zäher und

**0 046 970**

fester wurden. Man erhält unmittelbar hellbraune, niedrigviskose Produkte, die in Wasser sehr gut löslich sind und ohne weitere Reinigung in kosmetischen Präparaten eingesetzt werden können. Die Reaktionsgemische enthalten mindestens 75 % Zuckerester, während nach dem Stand der Technik nur Zuckertenside mit einem Gehalt von 30 bis 45 % erhalten werden konnten.

Wie bereits weiter oben ausgeführt, können die erfindungsgemäss hergestellten Partialester direkt mit wässerigem Natriumsulfit umgesetzt werden. Es entstehen in Wasser sehr gut lösliche viskose Substanzen, die wie die Ausgangsprodukte ausgeprägte Tensid- und Emulgatoreigenschaften besitzen. Darüber hinaus sind jedoch diese mit Natriumsulfit umgesetzten Partialester hervorragende Löslichkeitsvermittler, mit deren Hilfe es möglich ist, beispielsweise Parfümöle, unlösliche Bakterizide und andere kosmetische Hilfsstoffe in Wasser bzw. in Alkoholwassergemischen klar zu lösen. Als mindestens einfach ungesättigte Carbonsäuren mit einer Kettenlänge von $C_{10}$ bis $C_{25}$, vorzugsweise einer Kettenlänge von $C_{10}$ bis $C_{18}$, werden insbesondere Rizinolsäure, Ölsäure, Linolsäure, Palmitoleinsäure, Elaidinsäure sowie Undecylensäure verwendet. Anstelle der reinen Säuren können auch Fettsäuregemische, in denen eine der obengenannten ungesättigten Säuren angereichert ist, eingesetzt werden. Prinzipiell geeignet sind ungesättigte Fettsäuren im Jodzahlbereich zwischen 35 und 140. Zu den besonders bevorzugten Säuren zählen die Rizinolsäure die Undecylensäure. Bei Verwendung von Undecylensäure als ungesättigter Fettsäurekomponente werden mild bakterizid bzw. fungizid wirkende Verbindungen erhalten. Ihr besonderer Vorteil gegenüber gleich wirksamen bekannten Undecylensäureabkömmlingen besteht darin, dass sie frei von Aminostickstoff und Halogen und somit physiologisch absolut unbedenklich sind.

Als Di- oder Polyhydroxyalkane mit 2 oder 3 Kohlenstoffatomen oder Mono- und Disaccharide kommen insbesondere Äthylenglykol, Glycerin, sowie leicht zugängliche Zucker, wie Glucose, und Saccharose, in Frage. Prinzipiell können aber alle im technischen Masstab zugänglichen und physiologisch unbedenklichen mehrwertigen Alkohole und ihre Derivate eingesetzt werden.

Das erfindungsgemässe Verfahren zur Herstellung der gewünschtenfalls mit Natriumsulfit umgesetzten Partialester von Di- oder Polyhydroxyalkalen mit 2 oder 3 Kohlenstoffatomen oder Partialester von Mono- und Disacchariden mit den En-Addukten von Maleisäureanhydrid mit ungesättigten Carbonsäuren ist dadurch gekennzeichnet, daß man die En-Addukte von Maleinsäureanhydrid mit mindestens einfach ungesättigten Carbonsäuren mit der Kettenlänge $C_{10}$ bis $C_{25}$ und einer Jodzahl zwischen 35 und 140 ohne Lösungsmittel und ohne Katalysator bei Temperaturen von 150 bis 200°C innerhalb von 0,5 bis 5 h in Stickstoffatmosphäre herstellt, mit Wasser zu den Carbonsäuren hydrolysiert und danach bei 130 bis 140°C in an sich bekannter Weise mit den Di- oder Polyhydroxyalkanen verestert bis zu einer Säurezahl von höchstens 140, gewünschtenfalls bei 70 bis 100°C mit den Mono- oder Disacchariden umsetzt und gewünschtenfalls bei 70 bis 80°C nachträglich mit Natriumsulfit umsetzt. Für die Herstellung von Partialestern von Mono- und Disacchariden hat es sich als besonders günstig erwiesen, die En-Addukte von Maleinsäureanhydrid mit den ungesättigten Carbonsäuren zunächst zu hydrolisieren, dann mit Äthylenglykol zu verestern und anschliessend diesen Partialester mit dem Zucker umzuestern. Man erhält auf diese Art und Weise besonders reine und gut verträgliche Produkte, die unter schonendsten Bedingungen hergestellt unmittelbar für kosmetische Produkte verwendet werden können. Gewünschtenfalls wird der Partialester anschliessend mit Natriumsulfit bei Temperaturen von 70-80°C umgesetzt. Wie bereits weiter oben ausgeführt, ist diese Sulfierungsreaktion äusserst überraschend, da beispielsweise Saccharoserizinolester nicht mit Sulfit reagieren. Eine Bestimmung der Jodzahl der sulfierten Produkte ergibt, dass diese unverändert bleibt und somit die Doppelbindung in den ungesättigten Carbonsäuren offensichtlich erhalten geblieben ist.

Zur Durchführung des erfindungsgemässen Verfahrens wird die Fettsäure oder das Fettsäuregemisch mit Maleinsäureanhydrid im Verhältnis 1 : 1 bis 1 : 2 zusammengegeben und ohne Lösungsmittel und Katalysator auf 150 bis 200°C erhitzt. Die Reaktionszeit beträgt 0,5 bis 5 h. Der Anhydridring des Maleinsäureanhydrids öffnet sich in dem gebildeten Addukt unter milden Bedingungen, durch Wasseranlagerung, wobei mehrwertige Carbonsäuren in einem Säurezahlbereich von 200 bis 350 entstehen. Erwärmt men diese Carbonsäure mit temperaturunempfindlichen mehrwertigen Alkoholen auf 130 bis 140°C, so bilden sich bereits unter Wasserabspaltung die erfindungsgemässen Partialester. Je nach Veresterungsgrad weisen diese Substanzen Säurezahlen bis 140 auf. Zur Herstellung von Zuckerestern, insbesondere Saccharoseestern, werden vorzugsweise Äthylenglykolester im Molverhältnis 1 : 1 bis 1 : 0,5 auf 70 bis 100, vorzugsweise 85 bis 90°C erhitzt. Man wählt sie in Abhängigkeit der Säurezahl der Partialester so aus, dass am Ende der Reaktion keiner der Reaktionspartner in unreagierter Form zurückbleibt.

Bei niedrigen Tempereturen verläuft die Reaktion relativ langsam, bei Temperaturen über 100°C verfärben sich die erfindungsgemässen Saccharoseester missfarbig dunkel.

Für den Fall, dass man mit Natriumsulfit umgesetzte Partialester wünscht, wird anschliessend mit wässerigem Natriumsulfit bei Temperaturen zwischen 70 und 80°C umgesetzt, wobei im allgemeinen klar gelbe viskose Produkte mit hoher Oberflächenaktivität entstehen. Diese Bedingungen sind so schonend, dass keine inversion der Saccharose stattfindet.

Die erfindungsgemässen Produkte können besonders vorteilhaft als hautfreundliche, nichtionogene und/oder anionenaktive oberflächenaktive Substanzen in kosmetischen Präparaten verwendet werden. Sie können beispielsweise als Tenside in Shampoos und Emulgatoren in Reinigungscremen und Lotionen verwendet werden, erfindungsgemäss bereitete Shampoos haben den Vorteil, weder das Haar zu stark zu entfetten noch die Haarproteine zu denaturieren. Sie können aufgrund ihrer lösungsvermittelnden Eigenschaften sehr gut mit anderen typischen inhaltsstoffen von Kosmetika zusammen verarbeitet werden,

4

beispielsweise Pflanzenölen, Bakteriziden, desodorierenden Wirkstoffen und schwer löslichen Geruchsstoffen.

Mit Hilfe von Tensiden der erfindungsgemässen Art mit Undecylensäure als Ausgangsprodukt können Körperpflegemittel hergestellt werden, bei denen eine mild bakterizide und fungizide Wirkung besonders erwünscht ist, z. B. Intim- und Fusspflegemittel. Derartige Partialestertenside auf Undecylensäurebasis wirken mild aber signifikant gegen Bakterien und Pilze, wie sie z. B. im Intimbereich angesiedelt sind, aber auch, bekannterweise, das Verderben kosmetischer Präparate bewirken.

Daher ist ihre Verwendung als besonders hautschonendes Konservierungsmittel, von denen keinerlei physiologisch-schädigende Einflüsse zu erwarten sind, angezeigt.

In den nachfolgenden Beispielen wird die Erfindung näher erläutert.

**Beispiel 1**

298,45 g Eizinolsäure (1 mol) werden in einem Dreihalskolben mit 98 g (1 mol) Maleinsäureanhydrid ohne Lösungsmittel und ohne Katalysatoren, unter Rühren in Stickstoffatmosphäre bei 170-175°C innerhalb von 5 h umgesetzt.

Nach Beendigung der Reaktion wird das Reaktionsprodukt auf 60-70°C abgekühlt und mit 18 g Wasser versetzt.

Die Säurezahl der entstehenden mehrwertigen Carbonsäure beträgt 255. Die Säure wird anschliessend im gleichen Reaktionsgefäss mit 95 g Äthylenglykol bei 130-135°C unter 23,5 g Wasserabspaltung verestert, wobei ein Partialester mit der Säurezahl 80 entsteht.

Der Partialester wird mit 173,2 g Saccharose und 45,85 g Kaliumcarbonat bei 85-90°C unter Rühren in Stickstoffstrom innerhalb von 5 h umgesetzt, wobei 523 g eines hellbraunen, dickflüssigen Produktes entstehen. Der Rohester wird in 700 ml n-Butanol aufgenommen und dreimal mit 250 ml 7-%-iger wässeriger Natriumchloridlösung ausgewaschen und der ölige Rückstand in Chloroform aufgenommen. Der Chloroformextrakt wird abfiltriert und das Chloroform in einem Rotationsverdampfer von den 354,3 g Saccharoseester (67,7 % bezogen auf Gesamtreaktionsprodukt), der als hellbraune Flüssigkeit anfällt, getrennt.

Die Umesterung mit Monosacchariden, z. B. Glucose oder Mannose, erfolgt in analoger Weise.

**Kenndaten des Saccharoseesters:**

Oberflächenspannung: $38,1 \cdot 10^{-5}$ N/cm
Verseifungszahl: 287
pH (1 %-ige Lösung): 8,1

**Beispiel 2**

Es wird 1 kg Saccharoseester aus Rizinolsäure, Maleinsäureanhydrid und Zucker gemäss Beispiel 1 hergestellt.

Der Ester wird in einem Dreihalskolben, versehen mit Rührer und Rückflusskühler, mit 272 g Natriumsulfitheptahydrat, gelöst in 272 g Wasser, bei 70°C in 5 h sulfiert. Nach Beendigung der Reaktion erhält man 1530 g eines klaren gelben viskosen Produktes mit sehr guter Löslichkeit in Leitungswasser.

**Beispiel 3**

298,45 g Rizinolsäure (1 mol) werden mit 98 g Maleinsäureanhydrid (1 mol) gemäss Beispiel 1 umgesetzt und mit Äthylenglykol so weit verestert, dass ein Partialester mit der Säurezahl 30 bis 40 entsteht.

523 g der Partialester werden mit 177,8 g Saccharose und 75 g Natriummethylatlösung (25 %-ig) bei 85°C unter Rühren in Stickstoffstrom innerhalb von 5 h umgesetzt.

Anschliessend wird das Reaktionsgemisch im Rotationsverdampfer vom Methanol befreit, wobei 698 g eines hellbraunen rohen Saccharoseesters erhalten werden. Die Reinigung des rohen Zuckeresters gemäss Beispiel 1 ergibt 467,7 g (67 % bezogen auf Gesamtreaktionsprodukt) Saccharoseester.

**Kenndaten das Saccharoseesters:**

Oberflächenspannung: 39,3 · 10⁻⁵ N/cm
Verseifungszahl: 295
pH (1 %-ige Lösung): 8,0

**Beispiel 4**

298,45 g Rizinolsäure (1 mol) werden mit 98 g Maleinsäureanhydrid (1 mol) gemäss Beispiel 1 umgesetzt und mit Äthylenglykol verestert. Dabei entsteht ein Partialester mit einer Säurezahl um 10.

400 g dieses Partialesters werden mit 80 g Saccharose und 10 g Kaliumcarbonat gemäss Beispiel 1 umgesetzt. Nach 5-stündiger Reaktionszeit werden 467 g eines hellbraunen hochviskosen Produktes mit folgenden Kenndaten erhalten.

**Kenndaten des Saccharoseesters:**

Oberflächenspannung: 40,3 · 10⁻⁵ N/cm
Verseifungszahl: 283
pH (1 %-ige Lösung): 8,4

**Beispiel 5**

184,3 g Undecylensäure (1 mol) werden mit 98 g Maleinsäureanhydrid (1 mol) gemäss Beispiel 1 umgesetzt und mit Äthylenglykol verestert. Dabei entsteht ein Partialester mit einer Säurezahl um 100.

Der Partialester wird mit 252 g Natriumsulfitheptahydrat, gelöst in 300 g Wasser, bei 80°C in 1 h sulfiert, wobei 907 g einer klaren, hellbraunen, stark schäumenden Lösung mit bakterizider und fungizider Wirkung erhalten werden. Die Oberflächenspannung dieses Produktes beträgt 29,3 · 10⁻⁵ N/cm.

**Beispiel 6**

184,3 g Undecylensäure (1 mol) werden mit 98 g Maleinsäureanhydrid (1 mol) gemäss Beispiel 1 umgesetzt und mit 184,14 g Glycerin (2 mol) verestert, wobei 454 g eines hellgelben hochviskosen Partialesters mit sehr guter emulgierender sowie bakterizider und fungizider Wirkung erhalten werden.

Die folgenden Beispiele 7 bis 16 verdeutlichen Anwendungsmöglichkeiten der vorstehend beschriebenen Tenside zur Herstellung kosmetischer Präparate.

**Beispiel 7**

**Gesichtsreinigungsmilch**

| | |
|---|---|
| Verbindung nach Beispiel 3 | 10,0 |
| Cremophor® A 25 | 2,0 |
| (Äthoxylierter Fettalkohol-BASF) | |
| Cetylalkohol | 7,0 |
| Miglyol® 812 | 5,0 |
| (Triglycerid gesättigter Pflanzenfettsäuren) | |
| Konservierungsmittel | 0,2 |
| (Methyl-4-hydroxybenzoat-Na-Salz) | |
| H₂O | 68,8 |
| | ——— |
| | 100,0 |

6

**Beispiel 8**

**Tagescreme**

| | |
|---|---:|
| Verbindung nach Beispiel 1 | 8,0 |
| Cetylalkohol | 17,8 |
| Olivenöl | 7,0 |
| Paraffinöl | 6,0 |
| Hamamelis-Extrakt | 9,0 |
| Konservierungsmittel | 0,2 |
| (Methyl-4-hydroxybenzoat-Na-Salz) | |
| $H_2O$ | 52,0 |
| | 100,0 |

**Beispiele 9 und 10**

**Augenmakeup-remover**

| | | |
|---|---:|---:|
| Verbindung nach Beispiel 2 | 10,0 | 5,0 |
| Verbindung nach Beispiel 3 | - | 5,0 |
| Polyolfettsäureester | 5,0 | 5,0 |
| Carbopol® 934 (0,96 %-ig) | 79,8 | 79,8 |
| (Acrylsäuremischpolymerisat) | | |
| Konservierungsmittel | 0,2 | 0,2 |
| (Methyl-4-hydroxybenzoat- | | |
| Na-Salz) | | |
| $H_2O$ | 5,0 | 5,0 |
| | 100,0 | 100,0 |

**Beispiel 11**

**Öl/Wassercreme**

| | |
|---|---:|
| Verbindung nach Beispiel 4 | 4,0 |
| Cetylalkohol | 2,0 |
| Stearinsäure | 2,0 |
| Paraffinöl | 2,0 |
| 1,2-Propylenglykol | 2,0 |
| Glycerin | 1,5 |
| Konservierungsmittel | 0,2 |
| (Methyl-4-hydroxybenzoat-Na-Salz) | |
| $H_2O$ | 85,8 |
| Parfüm | 0,5 |
| | 100,0 |

**Beispiel 12**

**Deolotion**

| | |
|---|---|
| Grillocin HY 77 | 2,0 |
| (Deowirkstoff Zinkrizinoleatbasis - Grillo AG) | |
| Verbindung nach Beispiel 1 | 11,0 |
| Isopropylmyristat | 3,0 |
| Carbopol® 934 (0,96 %-ig in Wasser) | 83,8 |
| (Acrylsäuremischpolymerisat) | |
| Konservierungsmittel | 0,2 |
| (Methyl-4-hydroxybenzoat-Na-Salz) | |
| | 100,0 |

Das folgende Beispiel zeigt die lösungsvermittelnde Wirkung der nach Beispiel 2 hergestellten Tenside für wasserunlösliche Stoffe, die im kosmetischen Bereich verwendet werden.

**Beispiel 13**

**Solubilisierung**

| | | | | |
|---|---|---|---|---|
| a) Rosenöl | - | 1 | - | - |
| b) Bakterizid Irgasan® | | | | |
| DP 300 (Ciba) (2,4,4'-Trichlor-2'-hydroxydiphenyläther - Ciba) | - | - | - | 1 |
| c) Deowirkstoff Grillocin (Grillo AG)* | - | - | 1 | - |
| d) Menthol | 1 | - | - | - |
| Verbindung nach Beispiel 2 | 5 | 2 | 4 | 4 |
| H₂O | 94 | 97 | 95 | 95 |
| | 100,0 | 100,0 | 100,0 | 100,0 |
| Beurteilung | + | + + | + + | + + |
| klar löslich | | = + + | | |
| löslich mit leichter Trübung | | = + | | |

Unter den oben angegebenen Bedingungen sind die unter a bis d genannten Stoffe in Wasser unlöslich.

\* (Deowirkstoff Zinkrizinoleatbasis - Grillo AG)

**Beispiel 14**

**Antischuppenshampoo**

| | |
|---|---|
| Verbindung nach Beispiel 5 | 10,0 |
| Natriumlauryläthersulfat (28 %) | 50,0 |
| Polyquart H® | 5,0 |
| (Polyglykol/Polyamin-Kondensationsharz - Henkel KGaA) | |
| Comperlan KD® | 5,0 |
| (Kokosfettsäurediäthanolamid - Henkel KGaA) | |
| H₂O | 30,0 |
| | 100,0 |

**Beispiel 15**

**Intimpflege - Waschlösung**

| | |
|---|---|
| Verbindung nach Beispiel 5 | 10,0 |
| Rizinolsäuremonoäthanolamidsulfosuccinat | 15,0 |
| Natriumlauryläthersulfat (28 %) | 20,0 |
| Comperlan KD® | 3,0 |
| (Kokosfettsäurediäthanolamid - Henkel KGaA) | |
| Polyglykol 400 | 10,0 |
| Polyolfettsäureester | 7,0 |
| $H_2O$ | 35,0 |
| | 100,0 |

**Beispiel 16**

**Fussbad**

| | |
|---|---|
| Verbindung nach Beispiel 6 | 8,0 |
| Natriumlauryläthersulfat | 30,0 |
| 1,2-Propylenglykol | 2,0 |
| $H_2O$ | 60,0 |
| | 100,0 |

Die gute Haut- und Schleimhautverträglichkeit der erfindungsgemässen Partialester geht aus den folgenden Untersuchungen hervor. In der Tabelle 1 werden Ergebnisse des Augenreiztestes nach Draize für die erfindungsgemässen Produkte mit denen von bekannten, besonders hautfreundlichen anionischen Tensiden verglichen. Dabei wird auch auf die erheblich höheren Wirkstoffkonzentrationen bei den erfindungsgemässen Produkten besonders hingewiesen.

**Tabelle 1**

| Test Nr. | Produktbezeichnung | Konzentration (%) Wirksubstanz | Bewertungspunkte gemäss Draizescore | Bewertungsbereich |
|---|---|---|---|---|
| 1 | Verbindung nach Beispiel 1 | 100 | 15,6 | leicht rezeind |
| 2 | Verbindung nach Beispiel 2 | 65 | 1,8 | nicht reizend |
| 3 | Verbindung nach Beispiel 3 | 100 | 3,6 | nicht reizend |
| 4 | Verbindung nach Beispiel 4 | 100 | 4,2 | nicht reizend |
| 5 | Verbindung nach Beispiel 5 | 50 | 6,3 | nicht reizend |
| 6 | Talgkokosfettalkoholsulfat-Na-Salz | 40 | 24,3 | leicht reizend |
| 7 | Ammoniumlaurylsulfat | 35 | 12,9 | leicht reizend |
| 8 | Na- Lauryläthersulfat | 50 | 14,2 | leicht reizend |
| 9 | Na-Salz des Undecylensäuremono-äthanolamidsulfobernsteinsäure-halbesters | 50 | 19,6 | leicht reizend |

Die Durchführung des Draizetestes erfolgte gemäss den Bestimmungen des „Appraisal of the Safety of Chemicals in Food, Drug and Cosmetics", herausgegeben von den Food and Drug Officials of the United States, 1959.

Demnach gelten folgende Bewertungspunkte:

| | **Bewertungsbereiche** |
|---|---|
| 0 bis 10 | non-irritant |
| 11 bis 25 | slightly irritant |
| 16 bis 56 | moderately irritant |
| 57 bis 110 | severely irritant |

Die bakterizide und fungizide Wirksamkeit der Partialester auf Basis von Undecylensäure ergibt sich aus den folgenden Daten der Tabelle 2 (Suspensionsversuch mit der Substanz gemäss Beispiel 5, Konzentration 50 %).

**Tabelle 2**

| Testkeim | Escherichia coli | Staphylococcus aureus | Pseudomonas aeruginosa | Candida albicans |
|---|---|---|---|---|
| Anzahl der je Kultur eingeimpften Testkeime | 1 600 000 | 1 800 000 | 2 500 000 | 770 000 |
| *Einwirkungszeiten* | | | | |
| 2,5 min | - | - | - | - |
| 5 min | - | - | - | - |
| 10 min | - | - | - | - |
| 15 min | - | - | - | - |
| 30 min | - | - | - | - |
| 1 h | - | - | - | - |
| Blindwert mit physiologischer NaCl-Lösung | + + | + + | + + | + + |

Zeichenerklärung
- kein Wachstum
+ schwaches Wachstum
+ + starkes Wachstum

Der Test wurde in Anlehnung zu den Richtlinien für die Prüfung chemischer Desinfektionsmittel, herausgegeben von der Deutschen Gesellschaft für Hygiene und Mikrobiologie, durchgeführt.

*Testkeime*
*Escherichia coli ATCC 11229*
*Staphylococcus aureus ATCC 5638*
*Pseudomonas aeruginosa ATCC 15942*
*Candida albicans CBS 5703*

**Patentansprüche**

1. Gewünschtenfalls mit Natriumsulfit umgesetzte Partialester von Di- oder Polyhydroxyalkanen mit 2 oder 3 Kohlenstoffatomen oder Partialester von Mono- und Disacchariden mit den En-Addukten von Maleinsäureanhydrid an mindestens einfach ungesättigte Carbonsäuren mit einer Kettenlänge von $C_{10}$ bis $C_{25}$, erhältlich durch Umsetzung der Carbonsäuren mit Maleinsäureanhydrid ohne Lösungsmittel und ohne Katalysator bei Temperaturen von 150 bis 200°C innerhalb von 0,5 bis 5 h in Stickstoffatmosphäre, Hydrolyse der so erhaltenen En-Addukte und anschließende Veresterung mit den Di- oder Polyhydroxyalkanen bei 130 bis 140°C bis zu einer Säurezahl von höchstens 140, woraufhin gewünschtenfalls bei 70 bis 100°C mit den Mono- oder Disacchariden umgesetzt und gewünschtenfalls bei 70 bis 80°C nachträglich mit Natriumsulfit umgesetzt wird.

2. Verfahren zur Herstellung von mit gewünschtenfalls Natriumsulfit umgesetzten Partialestern von Di- oder Polyhydroxyalkanen mit 2 oder 3 Kohlenstoffatomen oder Partialestern von Mono- und Disacchariden mit den En-Addukten von Maleinsäureanhydrid an mindestens einfach ungesättigte Carbonsäuren mit der Kettenlänge $C_{10}$ bis $C_{25}$, dadurch gekennzeichnet, daß man die En-Addukte vom Maleinsäureanhydrid mit mindestens einfach ungesättigten Carbonsäuren mit der Kettenlänge $C_{10}$ bis $C_{25}$ und einer Jodzahl zwischen 35 und 140 ohne Lösungsmittel und ohne Katalysator bei Temperaturen von 150 bis 200°C innerhalb von 0,5 bis 5 h in Stickstoffatmosphäre herstellt, mit Wasser zu den Carbonsäuren hydrolysiert und danach bei 130 bis 140°C in an sich bekannter Weise mit den Di- oder Polyhydroxyalkanen verestert bis zu einer Säurezahl von höchstens 140, gewünschtenfalls bei 70 bis 100°C mit den Mono- oder Disacchariden umsetzt und gewünschtenfalls bei 70 bis 80°C nachträglich mit Natriumsulfit umsetzt.

3. Verfahren zur Herstellung von gewünschtenfalls mit Natriumsulfit umgesetzten Partialestern von Mono- oder Disacchariden mit den En-Addukten von Maleinsäureanhydrid mit mindestens einfach ungesättigten Carbonsäuren mit der Kettenlänge $C_{10}$ bis $C_{25}$ nach Anspruch 2, dadurch gekennzeichet, dass man das En-Addukt mit Äthylenglykol verestert.

4. Verwendung der gewünschtenfalls mit Natriumsulfit umgesetzten Partialester gemäss Anspruch 1 als hautfreundliche, nichtionogene und/ oder anionenaktive oberflächenaktive Substanzen in kosmetischen Präparaten.

5. Verwendung der gewünschtenfalls mit Natriumsulfit umgesetzten Partialester der Undecylensäure gemäss Anspruch 1 als hautfreundliche, mild bakterizide und fungizide nichtionogene und/oder anionenaktive oberflächenektive Substanzen in kosmetischen Präparaten.

## Claims

1. Partial esters, having optionally been reacted with sodium sulfite, of di- or polyhydroxyalkanes having 2 or 3 carbon atoms or partial esters of mono- and disaccharides with the ene-adducts of maleic anhydride to at least singly unsaturated carboxylic acids having a chain length of $C_{10}$ to $C_{25}$, obtainable by the reaction of the carboxylic acids with maleic anhydride in the absence of a solvent and in the absence of a catalyst at a temperature of from 150°C to 200°C within 0.5 to 5 h in a nitrogen atmosphere, hydrolysis of the ene-adducts thus obtained, and subsequent esterification with the di- or polyhydroxyalkanes at 130°C to 140°C to an acid number of at most 140, followed, if desired, by the reaction with the mono- or disaccharides at 70°C to 100°C and, if desired, by the subsequent reaction with sodium sulfite at 70°C to 80°C.

2. A process for preparing partial esters, having optionally been reacted with sodium sulfite, of di- or polyhydroxyalkanes having 2 or 3 carbon atoms or partial esters of mono- and disaccharides with the ene-adducts of maleic anhydride to at least singly unsaturated carboxylic acids having a chain length of $C_{10}$ to $C_{25}$, characterized in that the ene-adducts of maleic anhydride to at least singly unsaturated carboxylic acids having a chain length of from $C_{10}$ to $C_{25}$ and an iodine number of between 35 and 140 are prepared in the absence of a solvent and in the absence of a catalyst at a temperature of from 150°C to 200°C within 0.5 to 5 h in a nitrogen atmosphere, said ene-adducts are hydrolyzed with water to form carboxylic acids, the obtained acids are subsequently esterified with the di- or polyhydroxyalkanes at 130°C t 140°C to an acid number of at most 140 in a _per se_ known manner, followed, if desired, by the reaction with the mono- or disaccharides at 70°C to 100°C and, if desired, by the subsequent reaction with sodium sulfite at 70°C to 80°C.

3. The process for preparing partial esters, having optionally been reacted with sodium sulfite, of mono- and disaccharides with the ene-adducts of maleic anhydride to at least singly unsaturated carboxylic acids having a chain length of $C_{10}$ to $C_{25}$ according to claim 2, characterized in that the ene-adduct- is esterified with ethylene glycol.

4. Use of the partial esters, having optionally been reacted with sodium sulfite, according to claim 1 as skin-compatible nonionic and/or anion-active surface-active substances in cosmetic preparations.

5. Use of the partial esters, having optionally been reacted with sodium sulfite, of the undecylenic acid according to claim 1 as skin-compatible, mildly bactericidal and fungicidal nonionic and/or anion-active surface-active substances in cosmetic preparations.


## Revendications

1. Esters partiels, ayant éventuellement réagi sur du sulfite de sodium, de di- ou polyhydroxyalcanes ayant 2 ou 3 atomes de carbone ou esters partiels de mono- et disaccharides, avec les produits d'addition En de l'anhydride maléique sur des acides carboxyliques au moins une fois insaturés ayant une longueur de chaîne de $C_{10}$ à $C_{25}$, que l'on peut obtenir par réaction des acides carboxyliques sur de l'anhydride maléique sans solvant et sans catalyseur à des températures de 150 à 200°C, en 0,5 à 5 heures sous atmosphère d'azote, hydrolyse des produits d'addition. En ainsi obtenus, puis estérification avec les di- ou polyhydroxyalcanes à 130-140°C jusqu'à un indice d'acide maximal de 140, après quoi, si on le souhaite, on fait réagir à 70-100°C sur les mono- ou disaccharides et on fait éventuellement réagir après coup à 70-80°C sur du sulfite de sodium.

2. Procédé pour la préparation d'esters partiels, ayant éventuellement réagi sur du sulfite de sodium, de di- ou polyhydroxyalcanes ayant 2 ou 3 atomes de carbone ou d'esters partiels de mono- et disaccharides avec les produits d'addition En de l'anhydride maléique sur des acides carboxyliques au moins une fois insaturés ayant une longueur de chaîne de $C_{10}$ à $C_{25}$, caractérisé en ce qu'on prépare les produits d'addition En de l'anhydride maléique avec des acides carboxyliques au moins une fois insaturés ayant une longueur de chaîne de $C_{10}$ à $C_{25}$ et un indice d'iode compris entre 35 et 140, sans solvant et sans catalyseur, à des températures de 150 à 200°C, en 0,5 à 5 heures sous atmosphère d'azote, qu'on les hydrolyse à l'eau pour donner les acides carboxyliques, puis qu'on les estérifie à 130 - 140°C, d'une manière connue en soi, avec les di- ou polyhydroxyalcanes jusqu'à un indice d'acide maximal de 140, qu'on les fait reagir éventuellement a 70 - 100°C sur les mono- ou disaccharides et qu'on les fait éventuellement réagir après coup à 70-80°C sur du sulfite de sodium.

3. Procédé pour la préparation d'esters partiels, ayant éventuellement réagi sur du sulfite de sodium, de mono- ou disaccharides avec les produits d'addition En de l'anhydride maléique avec des acides carboxyliques au moins une fois insaturés et de longueur de chaîne $C_{10}$ à $C_{25}$ selon la revendication 2, caractérisé en ce qu'on estérifie le produit d'addition En avec de l'éthylèneglycol.

4. Emploi des esters partiels ayant éventuellement réagi avec du sulfite de sodium selon la revendication 1 comme substances tensioactives, non-ionogènes et/ou anioniques, favorables pour la peau, dans des préparations cosmétiques.

5. Emploi des esters partiels, ayant éventuellement réagi avec du sulfite de sodium, de l'acide undécylénique selon la revendication 1, comme substances tensioactives non-ioniques et/ou anioniques, favorables pour la peau, modérement bactéricides et fongicides, dans des préparations cosmetiques.